# EUROPÄISCHE PATENTSCHRIFT

(11) **EP 2 211 928 B1**
(45) Veröffentlichungstag und Bekanntmachung des Hinweises auf die Patenterteilung: **20.06.2012**
(21) Anmeldenummer: 08846963.0
(22) Anmeldetag: 04.11.2008
(51) Int. Cl.: A61M 1/00

(54) **PRÜFEINHEIT FÜR WUNDDRAINAGEAUFLAGEN**
TEST UNIT FOR WOUND DRAINAGE COVERINGS
UNITÉ DE CONTRÔLE DESTINÉE À DES PANSEMENTS POUR DRAINAGE

(30) Priorität: 08.11.2007 CH 17342007
(43) Veröffentlichungstag der Anmeldung: 04.08.2010
(73) Patentinhaber: Medela Holding AG, 6340 Baar (CH)
(72) Erfinder: LARSSON, Michael, CH-6300 Zug (CH); FURRER, Simon, CH-6454 Flüelen (CH)
(74) Vertreter: Clerc, Natalia
(86) Internationale Anmeldenummer: PCT/CH2008/000465
(87) Internationale Veröffentlichungsnummer: WO 2009/059444

(56) Entgegenhaltungen:
- EP-A- 0 117 351
- WO-A-00/61333
- WO-A-2005/099644
- GB-A- 2 305 610
- GB-A- 2 442 132
- JP-A- 2007 103 609
- US-B1- 6 182 956

## Beschreibung

### Technisches Gebiet

Die Erfindung betrifft eine Prüfeinheit für Wunddrainageauflagen.

### Stand der Technik

Es ist bekannt, grosse oder schlecht heilende Wunden mit einer Unterdruck-Drainagevorrichtung zu behandeln. Dies beschreibt beispielsweise WO 94/20041. Die Wunde wird dabei mit einer Abdeckung, beispielsweise einer Folie oder einer steifen Haube, überdeckt, so dass ein Wundraum entsteht. In den Wundraum wird von aussen ein Drainageschlauch eingeführt, welcher mit einer Saugpumpe verbunden ist, um Wundsekret aus der Wunde abzusaugen. Um den Wundraum zu füllen und insbesondere um den Unterdruck gleichmässig über die Wundoberfläche zu verteilen, wird eine Wundauflage auf die Wunde gelegt. Diese Wundauflage besteht üblicherweise aus einer Schaumstoffeinlage mit entsprechend geeignet ausgebildeten Poren. Diese Schaumstoffeinlage kann zugleich als Absorptionskörper für das Wundsekret dienen.

Entsprechende Drainagewundauflagen sind beispielsweise aus WO 2006/056294, US 7 070 584, EP 1 284 777 und EP 0 620 720 bekannt. Eine Drainagewundauflage mit einer Schaumstoffeinlage ausserhalb der luftdichten Deckschicht ist in WO 2006/052839 beschrieben. Komplizierter aufgebaute Wunddrainageauflagen sind beispielsweise in WO 03/086232 und US 2002/0065494 offenbart.

Es gibt somit eine Fülle von Vorschlägen, wie derartige Wunddrainageauflagen aufgebaut sein könnten. Es ist jedoch schwierig herauszufinden, welche Wunddrainageauflage bei welcher Wunde und mit welchem Saugdruck am besten einsetzbar ist.

WO 2005/099644 offenbart eine Prüfeinheit für wunddrainageauflagen und ein Flüssigkeitsreservoir.

### Darstellung der Erfindung

Es ist deshalb eine Aufgabe der Erfindung, eine Vorrichtung zu schaffen, welche eine einheitliche Prüfung und eine optimierte Anwendung von Wunddrainageauflagen unter möglichst praxisnahen Bedingungen erlaubt.

Diese Aufgabe löset ein Prüfsystem mit den Merkmalen des Patentanspruchs 1.

Das erfindungsgemässe Prüfsystem enthält eine Prüfeinheit, wobei die Prüfeinheit für Wunddrainageauflagen umfasst:
einen Grundkörper mit mindestens einem Hohlraum,
mindestens eine im Grundkörper verlaufende Zuführungsleitung, welche eine Aussenseite des Grundkörpers mit dem Hohlraum verbindet,

eine Oberfläche des Grundkörpers, welche als Auflagefläche zur Auflage von Wunddrainageauflagen und ihren Abdeckungen ausgebildet ist und
mehrere im Grundkörper verlaufende Kanäle, welche den Hohlraum mit der Auflagefläche verbinden,
wobei bei luftdicht zugedeckter Auflagefläche im Hohlraum und den Kanälen ein Unterdruck erzeugbar ist.

Der Hohlraum und die Kanäle simulieren die Wunde. Der Hohlraum simuliert im Wesentlichen das Wundbett und die Kanäle die Poren im Wundgrund.

Über die mindestens eine Zufuhrungsleitung lassen sich unterschiedlich zusammengesetzte Wundflüssigkeiten in die simulierte Wunde einbringen. Dabei kann gewählt werden, ob die Wundflüssigkeit kontinuierlich, in vorbestimmten Zeitabständen oder einmalig zugeführt wird. Die Auflagefläche erlaubt ein einfaches und schnelles Auflegen von zu testenden Wunddrainageauflagen. Diese Wunddrainageauflagen lassen sich von einer luftdurchlässigen selbsthaftenden Folie abdecken, welche auf die Auflagefläche geklebt wird. Sie lassen sich aber auch mit den herstellerseitig empfohlenen spezifischen Abdeckungen, wie beispielsweise starren Kappen, in der Prüfeinheit verwenden. In diesem Fall wird einfach die Kappe auf die Auflagefläche aufgeklebt, beispielsweise mittels einer luftdichten selbsthaftenden Folie.

Vorzugsweise weist der Grundkörper der Prüfeinheit eine planparallele Grundplatte, eine Zuleitungsplatte und eine dazwischen angeordnete Dichtplatte auf, wobei die Zuleitungsplatte die Kanäle aufweist und mindestens eine Ausnehmung zur Bildung des Hohlraums. Der Grundkörper ist somit relativ einfach aufgebaut und kostengünstig herstellbar. Er lässt sich auch einfach reinigen, da die Kanäle und die Ausnehmung bei auseinander geschraubtem Grundkörper gut zugänglich sind.

Ein weiterer Vorteil des derart ausgebildeten Grundkörpers ist, dass mehrere Zuleitungsplatten mit derselben Grundplatte und Zwischenplatte verwendet werden können. Dadurch kann die Prüfeinheit die verschiedensten Grössen und Anordnungen von Hohlräumen und Kanälen simulieren.

Das erfindungsgemässe Prüfsystem für Wunddrainageauflagen weist eine derartige Prüfeinheit auf. Ferner umfasst es mindestens ein Flüssigkeitsreservoir, welches mit der mindestens einen Zuführungsleitung verbindbar ist, und einen Drainagebehälter, welcher über eine Vakuumleitung und einen Vakuumanschluss mit der Auflagefläche verbindbar ist.

Das Prüfsystem kann mit unterschiedlichsten Saugpumpen betrieben werden, um auch deren Wirkung in der Wunddrainage zu berücksichtigen. Vorzugsweise wird es jedoch mit einer Pumpe verwendet, welche eine Steuer- und Auswertelektronik umfasst bzw. mit dieser verbindbar ist. Dadurch lassen sich Höhe des angelegten Unterdrucks, Zeitdauer des angelegten Unterdrucks, evtl. Druckänderungen oder Pulssequenzen und die zugeführte Flüssigkeit steuern bzw. dokumentieren. Natürlich wird auch die Durchflussmenge und Durchflussrate der abgesaugten Drainageflüssigkeit gemessen und aufgezeichnet und gegebenenfalls zusätzlich in der Auswerteelektronik bearbeitet.

Die erfindungsgemässe Prüfeinheit erlaubt unter anderem folgende Messmöglichkeiten:
- Messung des Zeitbedarfs bei vorgewähltem Vakuum, bis eine bestimmte Menge Flüssigkeit von der Test-Wunddrainageauflage aufgenommen ist;
- Messung des unterschiedlichen Verhaltens der Wunddrainageauflage bei unterschiedlich angelegtem Unterdruck (z.B. Höhe, Pulssequenz, Dauer);
- Vergleich verschiedener Flüssigkeiten mit unterschiedlichen Eigenschaften, wie beispielsweise Wasser, Sekret, Blut, bakteriell verseuchtem Blut, saurem oder basischem bakteriellem Medium, Kochsalzlösung;
- Prüfen der unterschiedlichen Verhaltensweisen der Wunddrainageauflage bei sektorieller Beaufschlagung der Prüfeinheit mit Flüssigkeiten;
- Prüfen des unterschiedlichen Verhaltens und des unterschiedlichen Sättigungsgrades der Wunddrainageauflage in ihren einzelnen Zonen, z.B. vom Rand zur Mitte.

Das erfindungsgemässe Prüfsystem erlaubt somit eine einheitliche Prüfung von bekannten Wunddrainageauflagen. Es ermöglicht es, diese optimierter einzusetzen. Ferner ist es ein unabdingbares Mittel bei der Entwicklung von neuen Wunddrainageauflagen und -abdeckungen wie auch bei der Entwicklung von neuen Saugpumpen und neuen Verfahren in der Wunddrainage.

Weitere vorteilhafte Ausführungsformen gehen aus den abhängigen Patentansprüchen hervor.

### Kurze Beschreibung der Zeichnungen

Im Folgenden wird der Erfindungsgegenstand anhand von bevorzugten Ausführungsbeispielen, welche in den beiliegenden Zeichnungen dargestellt sind, erläutert. Es zeigen:
- Figur 1: eine schematische Darstellung eines erfindungsgemässen Prüfsystems;
- Figur 2: einen Ausschnitt gemäss Figur 1 mit Prüfeinheit und Flüssigkeitsreservoirsystem;
- Figur 3: eine Explosionsdarstellung der Prüfeinheit gemäss Figur 1;
- Figur 4: eine Ansicht einer Zuleitungsplatte der Prüfeinheit von unten;
- Figur 5: eine Explosionsdarstellung der Prüfeinheit und des Flüssigkeitsreservoirsystems gemäss Figur 2;
- Figur 6a: eine Ansicht auf die Prüfeinheit in einer ersten Anwendung;
- Figur 6b: eine graphische Darstellung der gemessenen Volumenwerte der ersten Anwendung;
- Figur 7a: eine Ansicht auf die Prüfeinheit in einer zweiten Anwendung;
- Figur 7b: eine graphische Darstellung der gemessenen Volumenwerte der zweiten Anwendung;
- Figur 8a: eine Ansicht auf die Prüfeinheit in einer dritten Anwendung;
- Figur 8b: eine graphische Darstellung der gemessenen Volumenwerte der dritten Anwendung;
- Figur 9a: eine Ansicht auf die Prüfeinheit in einer vierten Anwendung;
- Figur 9b: eine graphische Darstellung der gemessenen Volumenwerte der vierten Anwendung;
- Figur 9c: eine graphische Darstellung der gemessenen Volumenwerte bei Verwendung von verschiedenen Wundabdeckungen;
- Figur 9d: eine graphische Darstellung der gemessenen Volumenwerte bei Verwendung von verschiedenen Wundflüssigkeiten;
- Figur 9e: eine graphische Darstellung der gemessenen Volumenwerte bei Verwendung von verschiedenen Unterdrücken;
- Figur 9f: eine graphische Darstellung der gemessenen Volumenwerte bei Ver- wendung von verschiedenen Unterdrücken;
- Figur 10a: eine Ansicht auf die Prüfeinheit in einer fünften Anwendung;
- Figur 10b: eine graphische Darstellung der gemessenen Volumenwerte der fünften Anwendung;
- Figur 11a: eine Ansicht auf die Prüfeinheit in einer sechsten Anwendung;
- Figur 11b: eine graphische Darstellung der gemessenen Volumenwerte der sechsten Anwendung;
- Figur 12a: eine Ansicht auf die Prüfeinheit in einer siebten Anwendung;
- Figur 12b: eine graphische Darstellung der gemessenen Volumenwerte der siebten Anwendung;
- Figur 13a: eine Ansicht auf die Prüfeinheit in einer achten Anwendung;
- Figur 13b: eine graphische Darstellung der gemessenen Volumenwerte der achten Anwendung;
- Figur 14a: eine Ansicht auf die Prüfeinheit in einer ersten Anordnung der Wundabdeckung;
- Figur 14b: eine Ansicht auf die Prüfeinheit in einer zweiten Anordnung der Wundabdeckung;
- Figur 14c: eine Ansicht auf die Prüfeinheit in einer dritten Anordnung der Wundabdeckung;
- Figur 14d: eine graphische Darstellung der gemessenen Volumenwerte gemäss den drei Anordnungen der Figuren 14a bis 14c;
- Figur 15a: eine Ansicht auf die Prüfeinheit mit einem Absaugbalken in einer ersten Form;
- Figur 15b: eine Ansicht auf die Prüfeinheit mit einem Absaugbalken in einer zweiten Form;
- Figur 15c: eine Ansicht auf die Prüfeinheit mit einem Absaugbalken in einer drit- ten Form und
- Figur 15d: eine graphische Darstellung der gemessenen Volumenwerte gemäss den drei Anordnungen der Figuren 15a bis 15c.

### Wege zur Ausführung der Erfindung

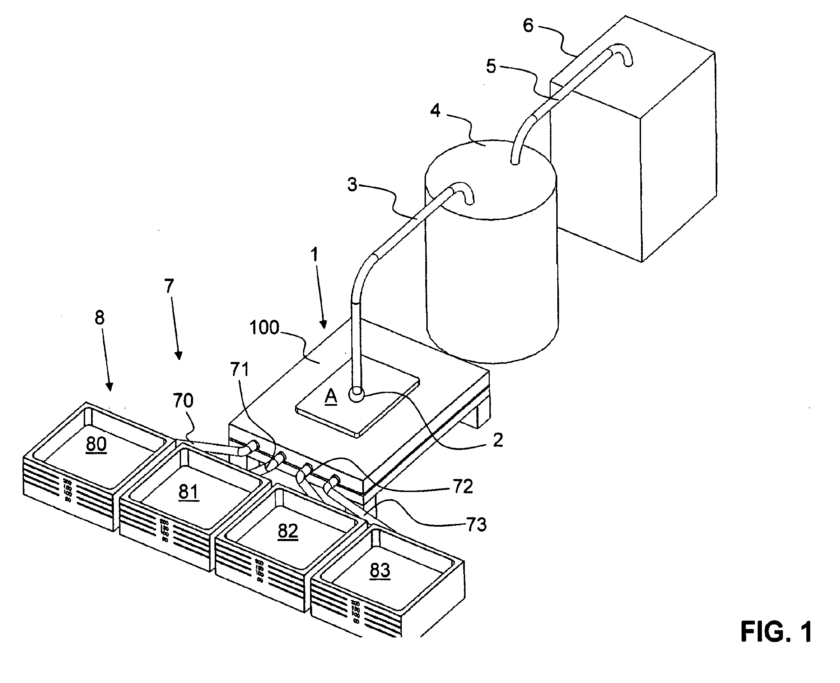

Figur 1 zeigt eine schematische Darstellung eines erfindungsgemässen Prüfsystems. Sie weist einen Grundkörper 1 einer Prüfeinheit, ein darauf angeordneter und luftdicht mit dem Grundkörper über eine Wundabdeckung A verbundener bzw. verbindbarer Vakuumanschluss 2, eine mit dem Vakuumanschluss 2 verbundene Drainageleitung 3, einen Drainagebehälter 4, in welchen die Drainageleitung 3 mündet, eine aus dem Drainagebehälter 4 herausführende Pumpleitung 5, und eine Saugpumpe 6 auf, welche mit der Pumpleitung 5 verbunden ist.

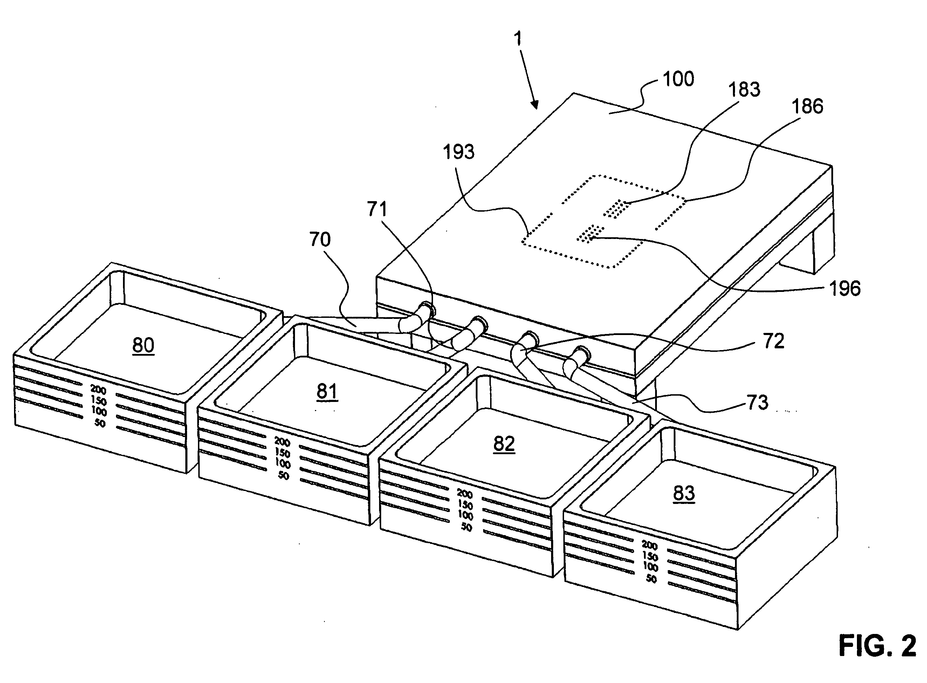

Der Grundkörper 1 ist ferner über ein Verbindungsleitungssystem 7, welches mindestens eine Verbindungsleitung 70, 71, 72, 73, aufweist, mit einem Flüssigkeitsreservoirsystem 8 verbunden. Dieses Flüssigkeitsreservoirsystem 8 weist mindestens ein Flüssigkeitsreservoir 80, 81, 82, 83 auf. Die Reservoirs 80, 81, 82, 83 verfügen vorzugsweise über eine Füllstandsanzeige, wie dies in Figur 2 erkennbar ist.

Der Grundkörper 1 weist, siehe Figuren 1 und 2, eine Auflagefläche 100 auf, welche vorzugsweise plan ausgebildet ist und mehrere ins Innere des Grundkörpers 1 führende Kanäle 183, 186, 193, 196 aufweist. Auf dieser Auflagefläche 100 und mindestens einen Teil der Kanäle 183, 186, 193, 196 überdeckend lässt sich eine zu prüfende Wundauflage D auflegen, mit einer zu prüfenden oder einer standardmässigen Wundabdeckung A überdecken und beide dicht mit dem Vakuumanschluss und dem Grundkörper 1 verbinden. Vorzugsweise wird hierfür eine selbsthaftende Folie verwendet.

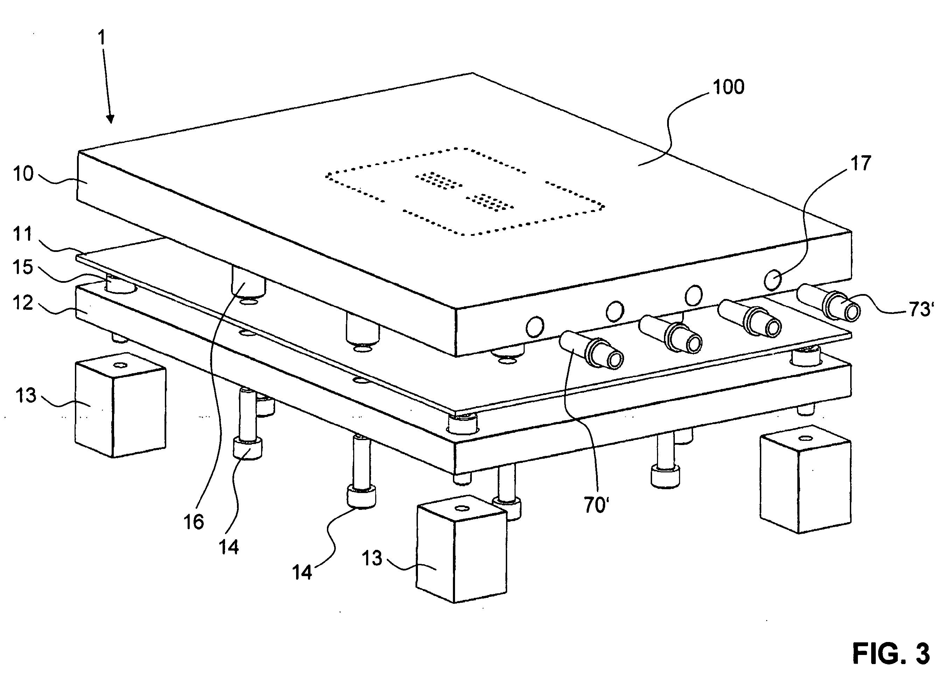

In den Figuren 3 bis 5 ist der Grundkörper 1 der Prüfeinheit detaillierter dargestellt. Er weist eine vorzugsweise planparallele Grundplatte 12, eine Zuleitungsplatte 10 und eine dazwischen angeordnete Dichtplatte 11 auf. Die Grundplatte 12 und die Zuleitungsplatte 10 sind vorzugsweise aus einem Kunststoff, insbesondere Plexiglas, oder einem Metall, insbesondere Stahl oder Aluminium, gefertigt. Die Dichtplatte 11 ist vorzugsweise aus einem flexiblen Dichtmaterial, insbesondere Silikon oder Gummi hergestellt.

Die Dichtplatte 11 ist ebenfalls vorzugsweise planparallel ausgebildet, wobei sie Durchführungsöffnungen aufweist. Durch diese Öffnungen sind Verbindungsschrauben 14 geführt, um die Grundplatte 12 mit der Zuleitungsplatte 10 luft- und flüssigkeitsdicht zu verschrauben. In der Zuleitungsplatte 10 sind hierfür Gewindelöcher vorgesehen oder Gewindebuchsen 16 fluchtend mit der unteren Oberfläche der Zuleitungsplatte 10 in diese eingelassen.

Vorzugsweise steht die Grundplatte 12 auf Füssen 13, welche sich beispielsweise ebenfalls über Befestigungsschrauben 15 mit dieser verschrauben lassen.

Die Grundplatte 12 weist vorzugsweise bis auf die für die Verbindung mit Füssen 13 und Zuleitungsplatte 10 keine Erhebungen oder Vertiefungen und auch keine inneren Bohrungen oder Kanäle auf. Die Zuleitungsplatte 10 ist vorzugsweise ebenfalls planparallel und dieselbe Form und Grundfläche aufweisend wie die Grundplatte 12 ausgebildet. Vorzugsweise sind beide durch je einen flachen Quader gebildet. Die Zuleitungsplatte 10 weist jedoch einerseits Ausnehmungen und andererseits Bohrungen auf.

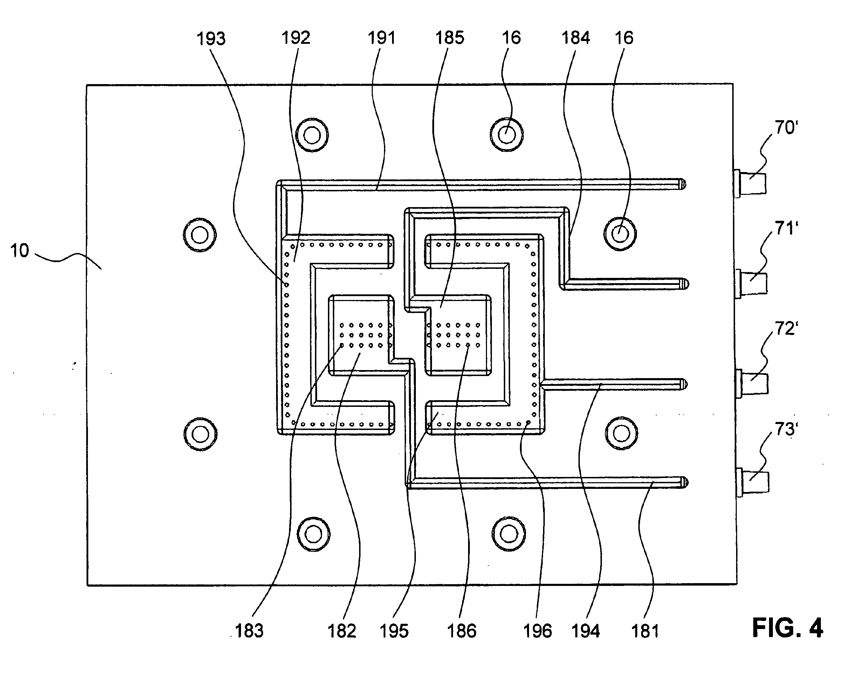

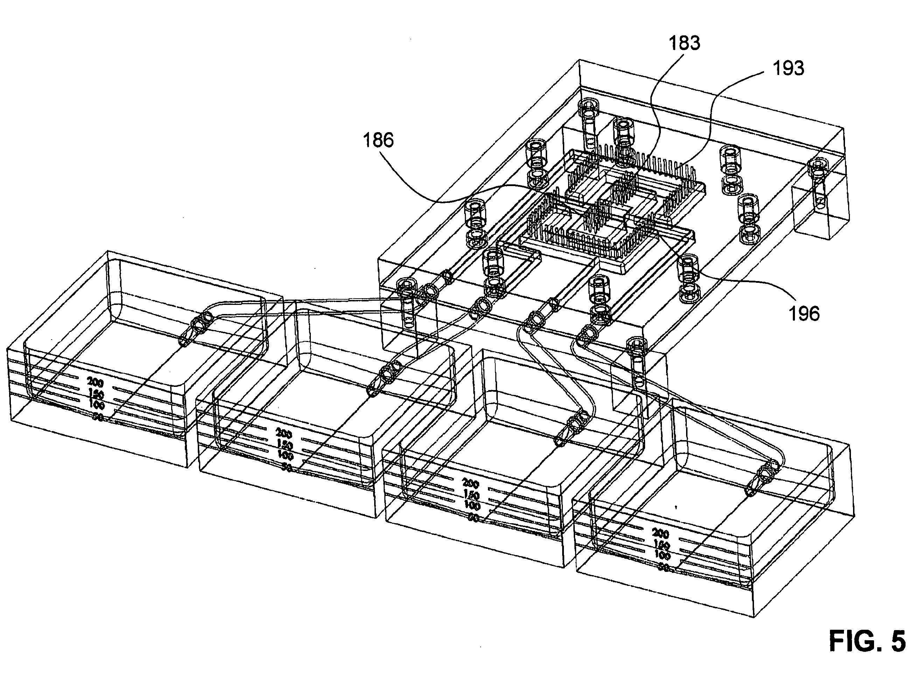

Wie in Figur 3 erkennbar ist, weist sie mindestens an einer, vorzugsweise genau an einer Stirnseite Bohrungen auf, welche Zuleitungsöffnungen 17 der in den Figuren 4 und 5 erkennbaren Zuführungsleitungen 181, 184, 191, 194 bilden. In diese Zuleitungsöffnungen 17 münden die oben erwähnten Verbindungsleitungen 70, 71. 72, 73. Hierfür sind vorzugsweise Anschlussstutzen 70', 71', 72', 73' vorhanden, welche in die Öffnungen 17 einsteckbar sind.

Erfindungsgemäss weist nun die Zuleitungsplatte 10 Ausnehmungen auf, welche nach oben, zur Auflagefläche 100 hin bis auf nachfolgend beschriebene Kanäle geschlossen und nach unten, zur Dichtplatte 11 und Grundplatte 12 hin offen ausgebildet sind. Diese Ausnehmungen werden durch die Dichtplatte 11 und die Grundplatte 12 zu voneinander vollständig getrennten Hohlräumen 182, 185, 192, 195 verschlossen. Sie können die unterschiedlichsten Formen aufweisen. Im hier dargestellten Beispiel weisen ein erster und zweiter Hohlraum 182, 185 einen gleich bleibenden rechteckförmigen Längsschnitt auf und sind einander benachbart, aber beabstandet zueinander angeordnet. Sie weisen hier dieselbe Grundfläche und vorzugsweise auch dieselbe Tiefe auf, so dass sie dasselbe Volumen aufweisen. Ein dritter und ein vierter Hohlraum 192, 195 sind jeweils einen der ersten und zweiten Hohlräume 182, 185 teilweise beabstandet umrahmend ausgebildet. Sie weisen hierfür einen ebenfalls vorzugsweise gleich bleibenden c-förmigen Längsschnitt auf. Auch sie weisen vorzugsweise dasselbe Volumen auf. Diese Hohlräume können jedoch auch andere Formen und Volumen aufweisen. Es können zudem mehr oder weniger als diese vier Hohlräume vorhanden sein. Sie können gemeinsam ein geometrisches Muster oder eine andere Anordnung in der Zuleitungsplatte 10 aufweisen. Des Weiteren können sie verschieden tief in Bezug auf die Auflagefläche 10 innerhalb der Zuleitungsplatte 10 angeordnet sein.

Diese Ausnehmungen sind über die oben genannten Zuführungsleitungen 181, 184, 191, 194 nach aussen offen ausgebildet. Auch diese Zuführungsleitungen 181, 184, 191, 194 sind in der Zuleitungsplatte 10 durch nach unten offene Nuten gebildet, welche erst im stirnseitigen Randbereich in geschlossene Röhren übergehen. Diese Nuten werden erst dank der Dichtplatte 11 und der Grundplatte 12 bis auf die Zuleitungsöffnungen 17 dicht verschlossen. Da keine Kavitäten gebildet werden müssen bzw. keine Bohrungen durchgeführt werden müssen, wird die Herstellung der Zuleitungsplatte erleichtert und sie lässt sich auch einfacher reinigen.

Diese Zuführungsleitungen 181, 184, 191, 194 können gleich oder unterschiedlich lang ausgebildet sein. Sie verlaufen vorzugsweise parallel zur Auflagefläche 100, so dass die Flüssigkeitszufuhr parallel zur Fläche der Wundauflage erfolgt. Vorzugsweise führt je eine Zuführungsleitung zu je einem Hohlraum und je eine Zuführungsleitung zu je einer Zuleitungsöffnung. Sie können sich jedoch auch verästeln und mehrere Hohlräume bedienen bzw. ein Hohlraum kann über mehrere Zuführungsleitungen verfügen. Vorzugsweise weisen alle Zuführungsleitungen denselben inneren Durchmesser auf. Sie können jedoch ebenfalls unterschiedliche Durchmesser aufweisen.

Von den Hohlräumen 182, 185, 192, 195 führen Kapillaren oder Kanäle 183, 186, 193, 196 nach aussen zur Auflagefläche 100. Jeder Hohlraum weist mehrere derartige Kanäle auf. Die Kanäle desselben Hohlraums können denselben oder unterschiedliche innere Durchmesser aufweisen. Desgleichen können Kanäle unterschiedlicher Hohlräume dieselben oder unterschiedliche Durchmesser aufweisen. Sie verlaufen vorzugsweise in senkrechter Richtung zur Auflagefläche 100, sie können jedoch auch in einem Winkel dazu verlaufen. Die Kanäle eines Hohlraums bilden auf der Auflagefläche vorzugsweise ein geometrisches Muster, wobei dieses je nach Hohlraum unterschiedlich ausgebildet sein kann. Vorzugsweise sind die Kanäle möglichst gleichmässig über die Grundfläche oder zumindest über einen Bereich des jeweiligen Hohlraums verteilt.

Die Hohlräume weisen vorzugsweise ein Volumen von 2 cm³ bis 4 cm³ mm auf. Die Kanäle sind vorzugsweise 3 mm bis 8 mm lang und weisen einen inneren Durchmesser von vorzugsweise 1 mm bis 1.5 mm auf. Die Zuführungsleitungen weisen vorzugsweise einen inneren Durchmesser von 2 mm bis 4 mm auf.

In einer bevorzugten Ausführungsform ist die Prüfeinheit heizbar, so dass die Temperaturen des Patienten simuliert werden können.

Dank dieser Prüfeinheit und diesem Prüfsystem lassen sich nun Drainageapplikationen testen. So lassen sich unterschiedliche Testflüssigkeiten von den Flüssigkeitsreservoirs gezielt in einzelne Hohlräume einbringen. Diese Testflüssigkeiten können Wundflüssigkeiten oder Behandlungsflüssigkeiten simulieren.

Auf die Auflagefläche lassen sich unterschiedliche Wundauflagen (dressing) auflegen und mit unterschiedlichen Wundabdeckungen abdecken. Des Weiteren lassen sich unterschiedlich geformte Vakuumanschlüsse (Drains) verwenden, welche sich zudem an unterschiedlichen Stellen in Bezug auf die Wundauflage und die gerade gefüllten Hohlräume anordnen lassen. Dank unterschiedlich geformter und unterschiedlich grossen Hohlräumen lassen sich verschiedene Wundbettarten simulieren. Des Weiteren kann das Verhalten derselben Wundauflagen, -abdeckungen und Drains mit unterschiedlichen Saugpumpen, unterschiedlichen Unterdrücken, Saugsequenzen und unterschiedlicher Drainagedauer getestet werden.

In den nachfolgend beschriebenen Figuren sind derartige Anwendungen gezeigt. Diese sind lediglich beispielhaft und nicht abschliessend zu verstehen:

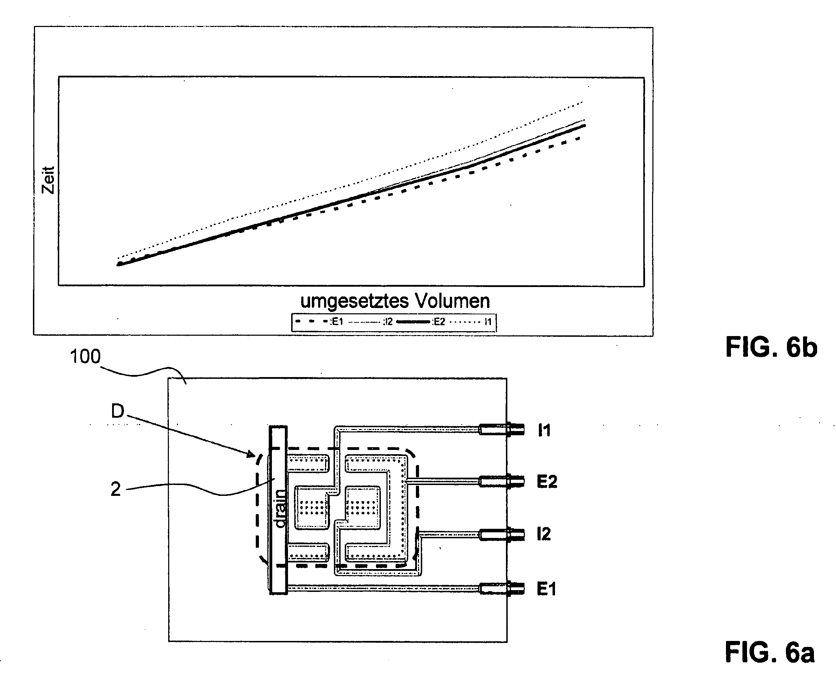

Figur 6a zeigt einen Vakuumanschluss (Drain) 2 in Form eines rechteckförmigen Balkens mit mehreren gleichmässig über seine Länge verteilten Saugöffnungen, welcher über einer rechteckförmigen Wundauflage D angeordnet ist. Diese Wundauflage D überdeckt den gesamten Kanalbereich der Auflagefläche 100. Über alle Zuleitungsöffnungen, hier mit E1, E2, I1 und I2 bezeichnet, wird den vier Hohlräumen nacheinander dieselbe Wundflüssigkeit zugeführt. In diesem Beispiel wird während des Absaugens keine Flüssigkeit mehr hinzugeführt. In anderen Beispielen wäre dies jedoch möglich. Jede einzelne Zuführung wird mit Unterdruck beaufschlagt und das Fliessverhalten gemessen. Dabei wird für alle vier Hohlräume nacheinander dieselbe Saugsequenz angewendet (d.h. unter anderem Dauer, Höhe des Unterdrucks, evtl. Variationen des Drucks während des Saugvorgangs).

Die Figur 6b zeigt die gemessenen Werte. Die y-Achse zeigt die Zeit, die x-Achse das bei der Absaugung umgesetzte Volumen. Dieses Volumen wird vorzugsweise im Drainagebehälter gemessen. Wie erkennbar ist, spielt es somit eine Rolle, in welchem Abstand der Drain 2 zum abgesaugten Hohlraum angeordnet ist.

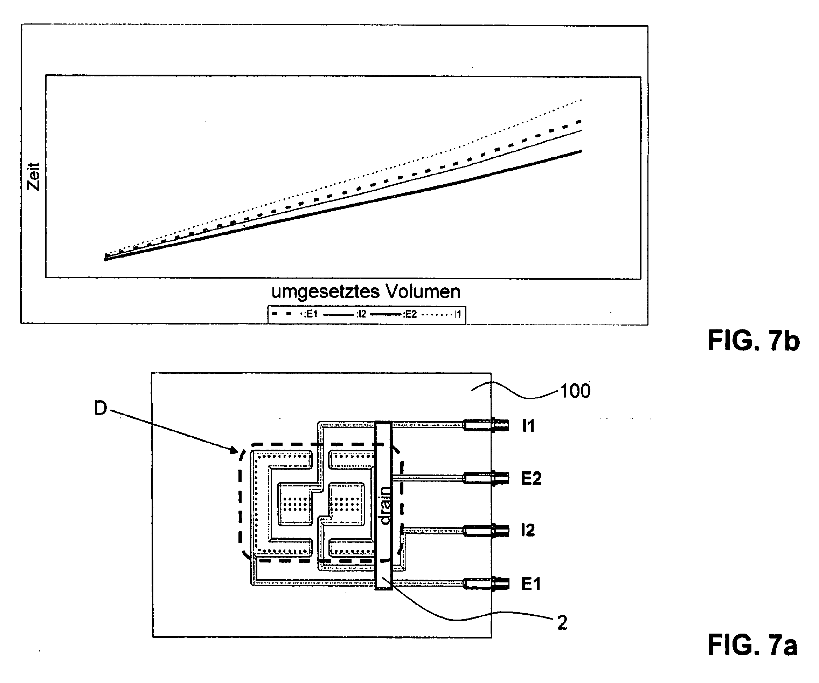

In Figur 7a wurde gleich vorgegangen und derselbe Drain 2, dieselbe Wundauflage D und dieselbe Testflüssigkeit verwendet, wobei hier der Drain 2 am gegenüberliegenden Ende der Hohlkammern angeordnet wurde.

In Figur 7b wurden wieder die gemessenen Werte festgehalten.

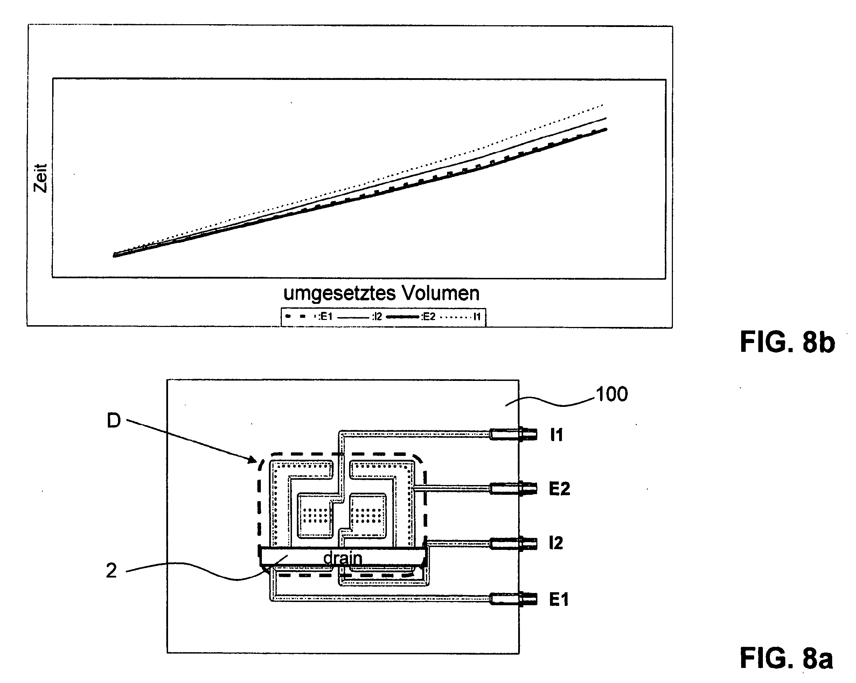

Im Beispiel gemäss den Figuren 8a und 8b wurde wieder gleich vorgegangen, wobei hier der Drain 2 im unteren Bereich angeordnet ist.

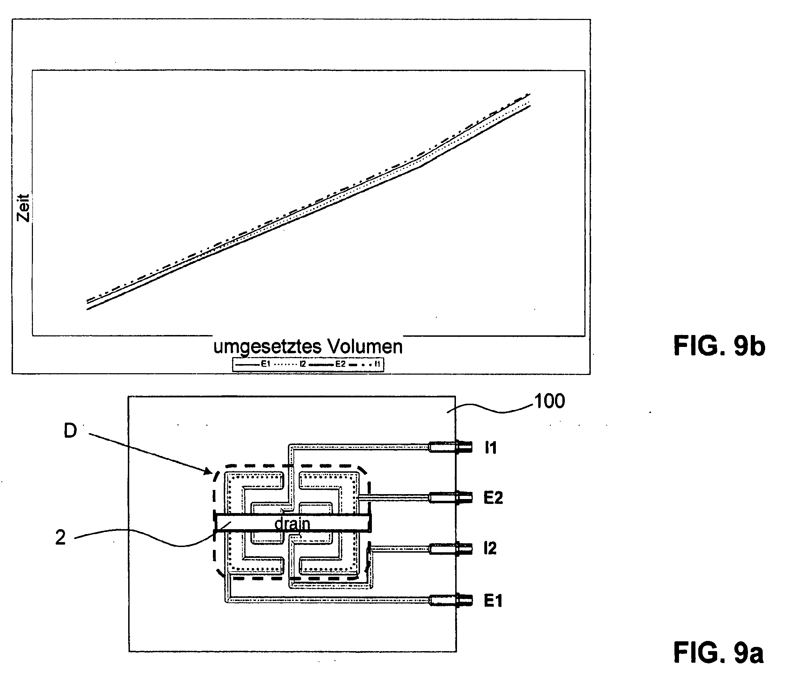

Im Beispiel gemäss den Figuren 9a und 9b ist erkennbar, dass bei gleichem Vorgehen wie in den anderen Beispielen, aber bei Platzierung des Drains 2 über der Mitte der Hohlkammern, am wenigsten Schwankungen beim Verhalten der vier Hohlkammern auftreten. In den folgenden Figuren wird deshalb nochmals diese Anordnung verwendet, um andere Parameter zu variieren.

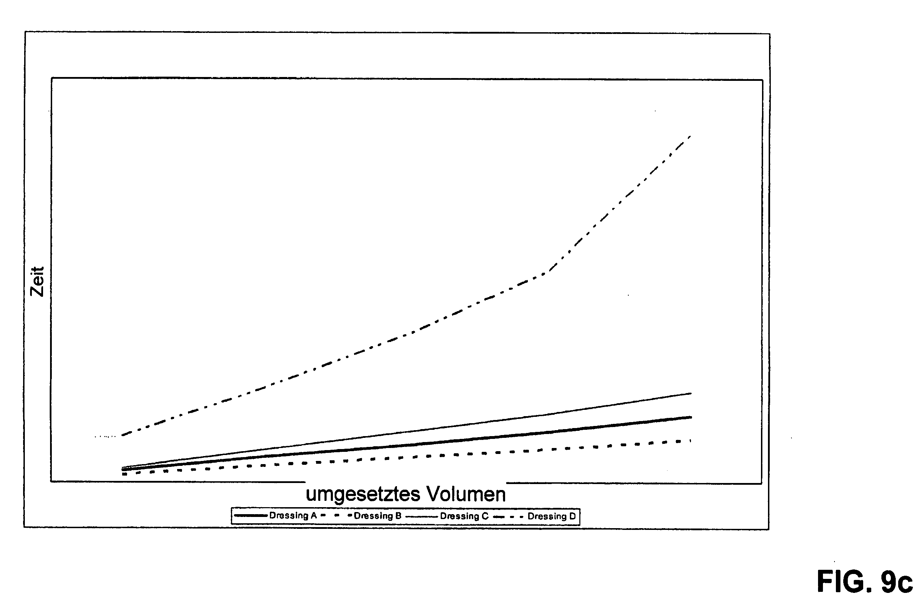

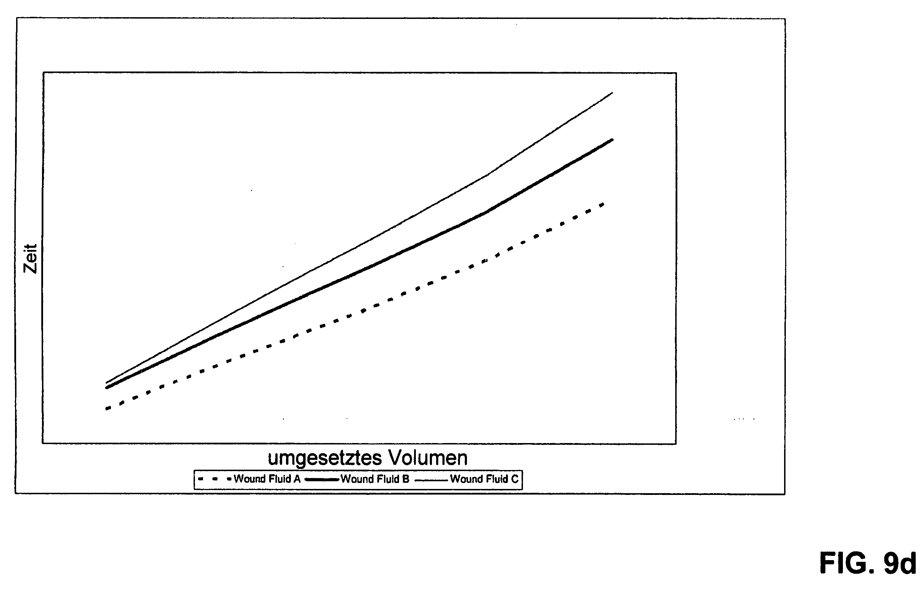

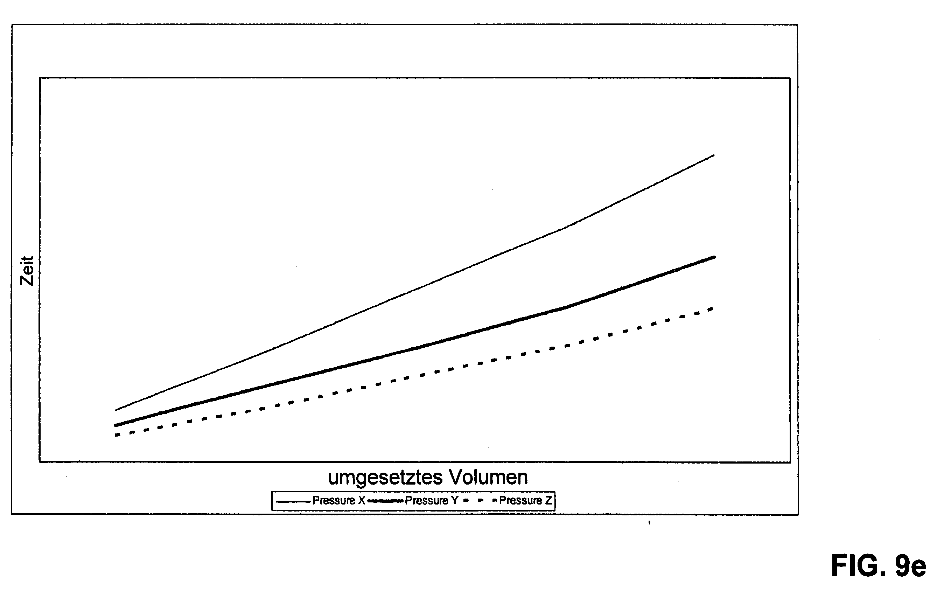

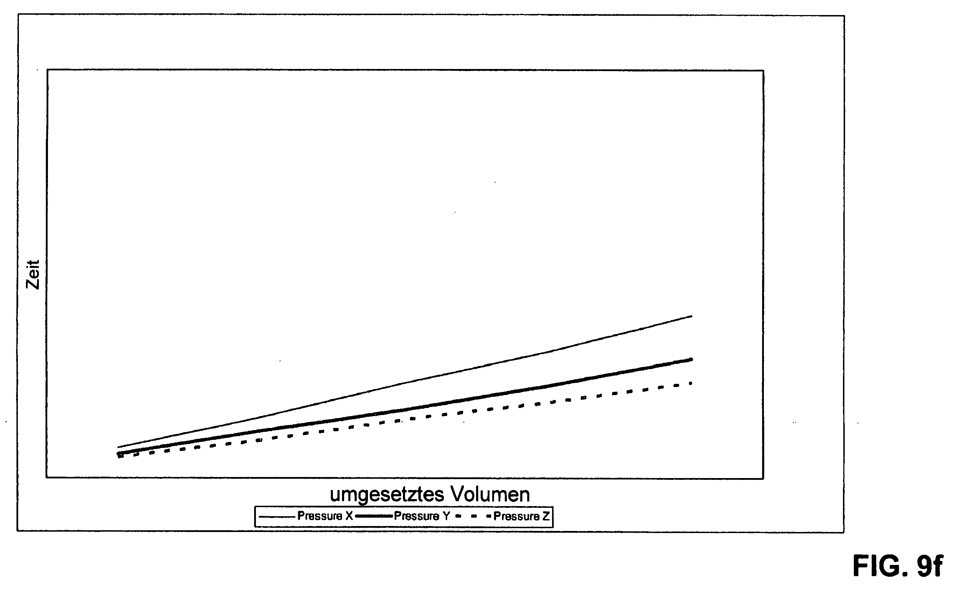

So wurden bei der Messung, welche in Figur 9c dargestellt ist, nacheinander vier unterschiedliche Wundauflagen (dressings), aber mit derselben Testflüssigkeit und derselben Saugsequenz verwendet. In Figur 9d wurden mit derselben Wundauflage und Saugsequenz nacheinander vier unterschiedliche Testflüssigkeiten zugeführt. Bei Figur 9e wurden bei gleich bleibender Wundauflage und gleich bleibender Testflüssigkeit unterschiedliche Saugsequenzen eingesetzt. Bei Figur 9f wurde eine andere Wundauflage aber dieselbe Testflüssigkeit verwendet. Auch hier wurde die Saugfrequenz variiert.

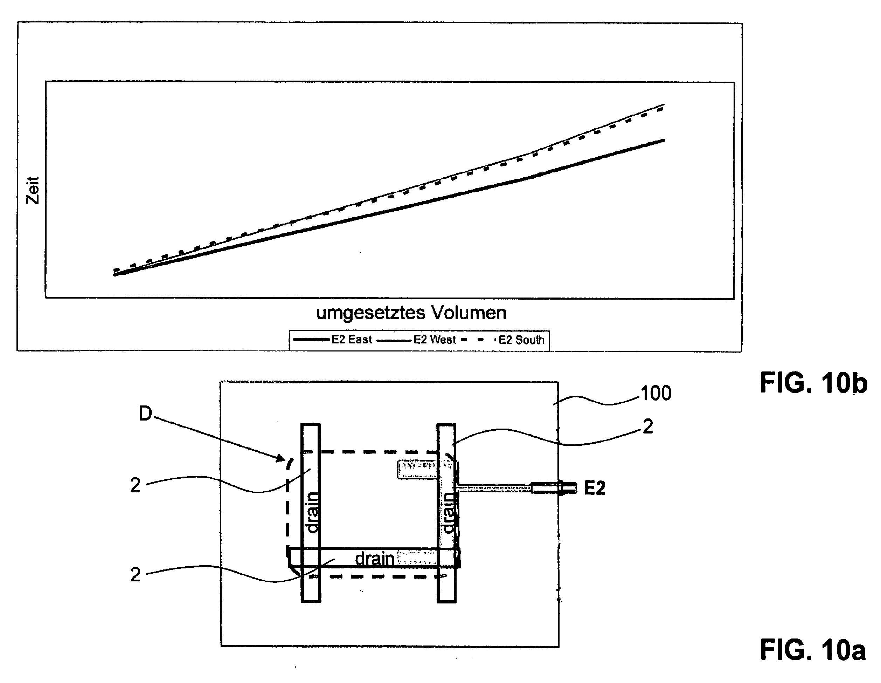

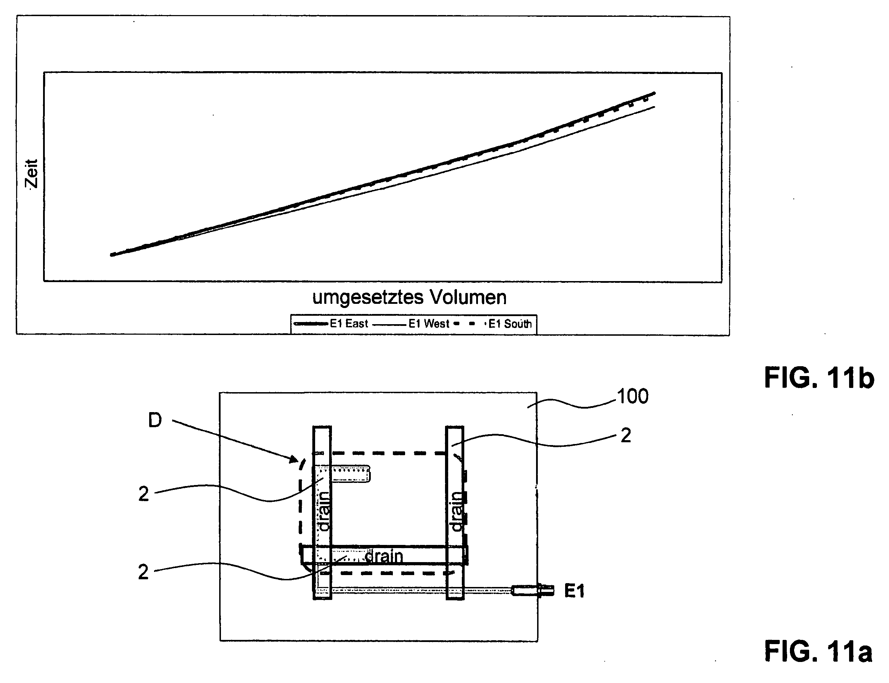

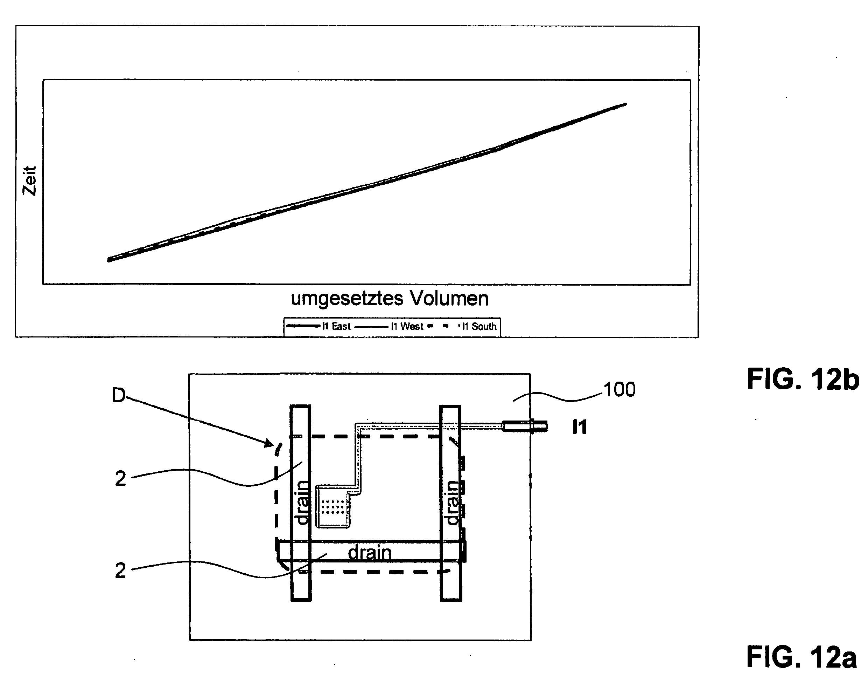

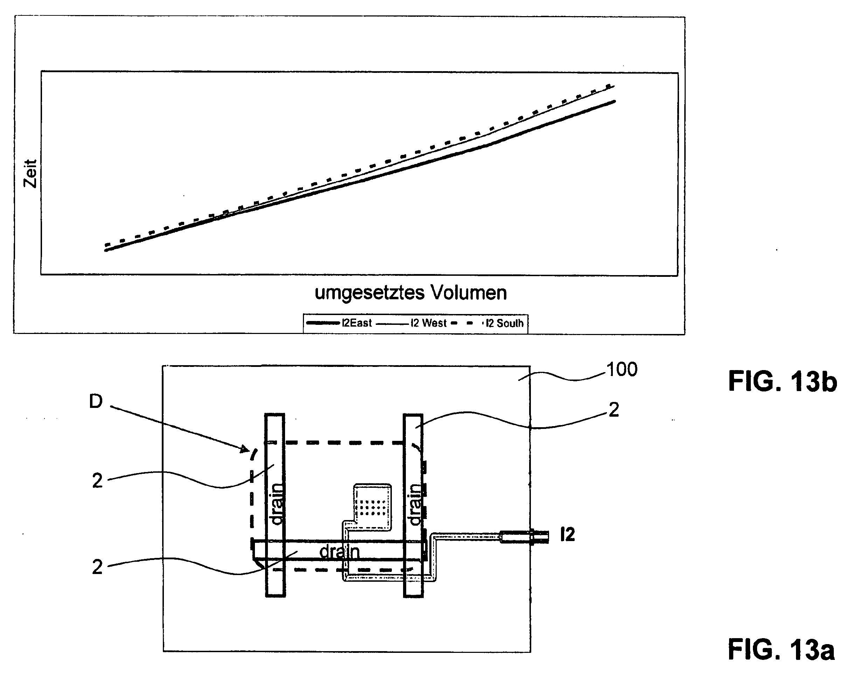

In den Figuren 10a und 10b ist ein Experiment dargestellt, bei welchem nacheinander drei an verschiedenen Stellen angeordnete Drains 2, aber immer derselbe Hohlraum verwendet wurden. Die Figuren 11 a und 11b zeigen das Experiment bei Verwendung eines anderen Hohlraums als in oben genanntem Beispiel. Dasselbe gilt für die Figuren 12a und 12b bzw. 13a und 13b.

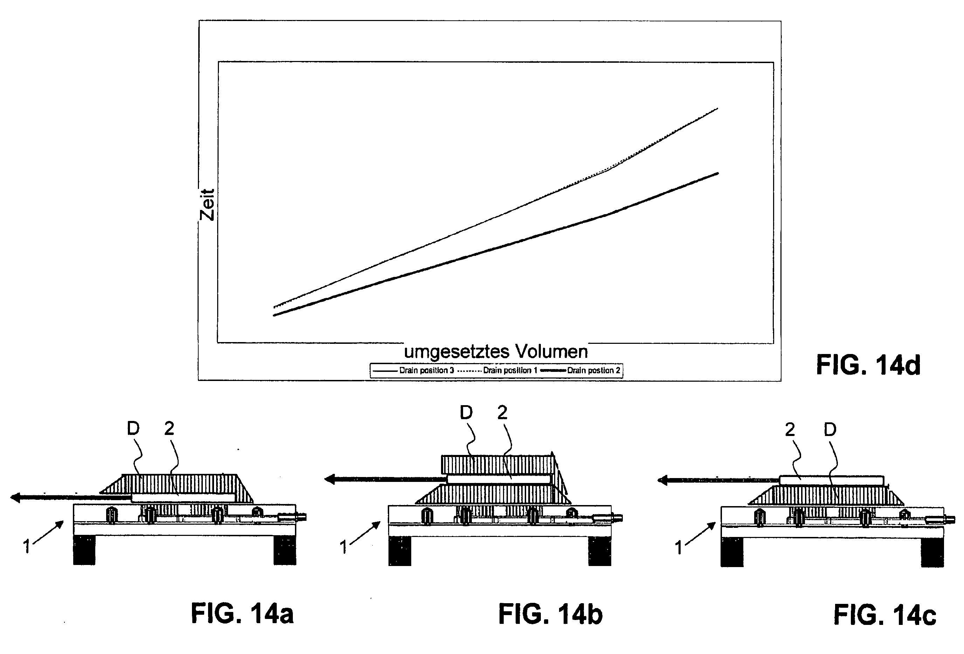

In Figur 14a ist der Drain 2 unterhalb der Wundauflage D angeordnet. In Figur 14b ist er innerhalb der Wundauflage D angeordnet und in Figur 14c oberhalb. Die Messergebnisse sind in Figur 14d dargestellt.

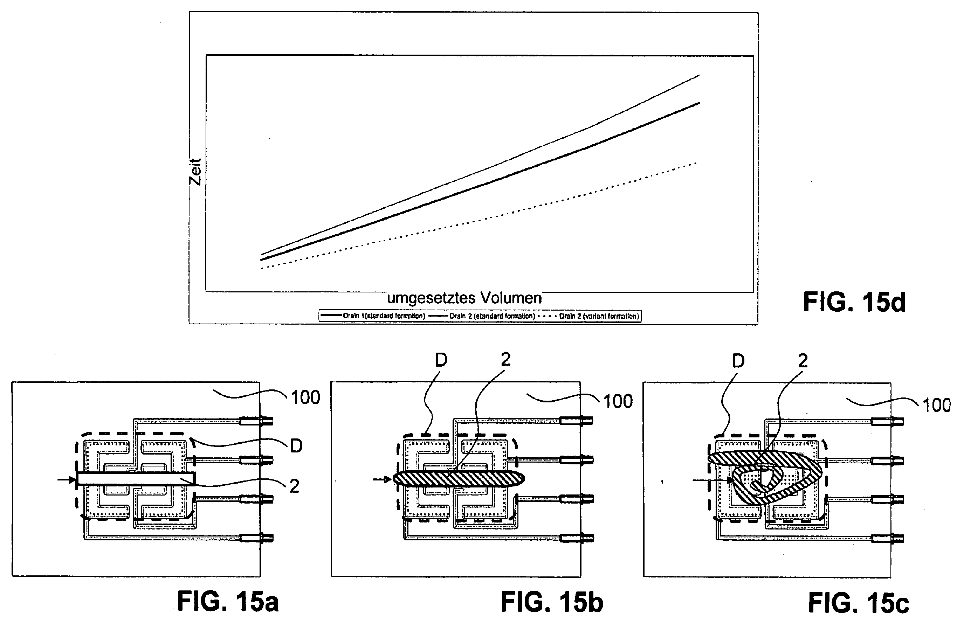

In den Figuren 15a bis 15c werden unterschiedlich geformte Drains 2 verwendet. Das Messergebnis ist wiederum in Figur 15d dargestellt.

Wie anhand dieser Beispiele erkennbar ist, lassen sich Wunddrainageapplikationen mit einfachen und kostengünstigen Mitteln auf unterschiedlichste Art und Weise testen.

### Bezugszeichenliste

- 1: Grundkörper der Prüfeinheit
- 10: Zuleitungsplatte
- 100: Auflagefläche
- 11: Dichtplatte
- 12: Grundplatte
- 13: Fuss
- 14: Verbindungsschraube
- 15: Befestigungsschraube
- 16: Gewindebuchse
- 17: Zuleitungsöffnung

- 181: erste Zuführungsleitung
- 182: erster Hohlraum
- 183: erste Kanäle

- 184: zweite Zuführungsleitung
- 185: zweiter Hohlraum
- 186: zweite Kanäle

- 191: dritte Zuführungsleitung
- 192: dritter Hohlraum
- 193: dritte Kanäle

- 194: vierte Zuführungsleitung
- 195: vierter Hohlraum
- 196: vierte Kanäle

- 2: Vakuumanschluss

- 3: Drainageleitung

- 4: Drainagebehälter

- 5: Pumpleitung

- 6: Saugpumpe

- 7: Verbindungsleitungsleitungssystem
- 70: erste Verbindungsleitung
- 70': erster Anschlussstutzen
- 71: zweite Verbindungsleitung
- 71': zweiter Anschlussstutzen
- 72: dritte Verbindungsleitung
- 72': dritter Anschlussstutzen
- 73: vierte Verbindungsleitung
- 73': vierter Anschlussstutzen

- 8: Flüssigkeitsreservoirsystem
- 80: erstes Flüssigkeitsreservoir
- 81: zweites Flüssigkeitsreservoir
- 82: drittes Flüssigkeitsreservoir
- 83: viertes Flüssigkeitsreservoir

- A: Wundabdeckung
- D: Wundauflage

## Patentansprüche

1. Prüfsystem für Wunddrainageauflagen mit mindestens einem Flüssigkeitsreservoir (8) und mit einer Prüfeinheit mit mindestens einer Zuführungsleitung (181, 184, 191, 194), wobei das mindestens eine Flüssigkeitsreservoir (8) mit der mindestens einen Zuführungsleitung (181, 184, 191, 194) verbindbar ist, **dadurch gekennzeichnet, dass** die Einheit umfasst:
einen Grundkörper (1) mit mindestens einem Hohlraum (182, 185, 192, 195);
mindestens eine im Grundkörper (1) verlaufende Zuführungsleitung (181, 184, 191, 194), welche eine Aussenseite des Grundkörpers (1) mit dem Hohlraum (182, 185, 192, 195) verbindet,
eine Oberfläche des Grundkörpers (1), welche als Auflagefläche (100) zur Auflage von Wundauflagen (D) und ihren Wunddrainageabdeckungen ausgebildet ist
und
mehrere im Grundkörper (1) verlaufende Kanäle (183, 186, 193, 196), welche den Hohlraum (182, 185, 192, 195) mit der Auflagefläche (100) verbinden,
wobei bei luftdicht zugedeckter Auflagefläche (100) im Hohlraum (182, 185, 192, 195) und den Kanälen (183, 186, 193, 196) ein Unterdruck erzeugbar ist und dass das Prüfsystem ferner einen Drainagebehälter (4) aufweist, welcher über eine Drainageleitung (3) und einen Vakuumanschluss (2) mit der Auflagefläche (100) verbindbar ist.

2. Prüfsystem, nach Anspruch 1, wobei der Grundkörper (1) mehrere Hohlräume (182, 185, 192, 195) aufweist, welche über voneinander getrennte Zuführungsleitungen (181, 184, 191, 194) mit mindestens einer Aussenseite des Grundkörpers (1) verbunden sind.

3. Prüfsystem nach einem der Ansprüche 1 oder 2, wobei die Auflagefläche (100) plan ausgebildet ist.

4. Prüfsystem nach einem der Ansprüche 1 bis 3, wobei mehrere Hohlräume (182, 185, 192, 195) vorhanden sind, welche verschieden grosse Volumen aufweisen.

5. Prüfsystem nach einem der Ansprüche 1 bis 4, wobei die Kanäle (183, 186, 193, 196) geradlinig im Grundkörper (1) verlaufen.

6. Prüfsystem nach einem der Ansprüche 1 bis 5, wobei die Kanäle (183, 186, 193, 196) einen gleich bleibenden Innendurchmesser aufweisen.

7. Prüfsystem nach einem der Ansprüche 1 bis 6, wobei die einem gemeinsamen Hohlraum (182, 185, 192, 195) zugeordneten Kanäle (183, 186, 193, 196) denselben Innendurchmesser aufweisen.

8. Prüfsystem nach einem der Ansprüche 1 bis 7, wobei die Kanäle (183, 186, 193, 196) eines gemeinsamen Hohlraums (182, 185, 192, 195) einen anderen Innendurchmesser aufweisen als die Kanäle (183, 186, 193, 196) eines anderen Hohlraums (182, 185, 192, 195).

9. Prüfsystem nach einem der Ansprüche 1 bis 8,
wobei der Grundkörper (1) eine Grundplatte (12), eine Zuleitungsplatte (10) und eine dazwischen angeordnete Dichtplatte (11) aufweist,
wobei die Grundplatte (12) planparallel ausgebildet ist und
wobei die Zuleitungsplatte (10) folgendes aufweist:
- die mindestens eine Zuführungsleitung (181,184,191, 194),
- mindestens eine Ausnehmung zur Bildung des mindestens einen Hohlraums (182, 185, 192, 195) und
- die Kanäle (183, 186, 193, 196).

10. Prüfsystem nach einem der Ansprüche 1 bis 9, wobei die mindestens eine Zuführungsleitung (181, 184, 191, 194) in einer Ebene senkrecht zu den Kanälen (183, 186, 193, 196) verläuft.

11. Prüfsystem nach einem der Ansprüche 1 bis 10, wobei mehrere Zuführungsleitungen (181, 184, 191, 194) vorhanden sind, welche in eine gemeinsame Stirnseite des Grundkörpers (1) münden.

12. Prüfsystem nach einem der Ansprüche 1 bis 11, wobei ein Vakuumanschluss (2) vorhanden ist, welcher über die Auflagefläche (100) applizierbar ist.

13. Prüfsystem nach einem der Ansprüche 1 bis 12, wobei es ferner eine Saugpumpe (6) umfasst.

14. Prüfsystem nach einem der Ansprüche 1 bis 13, wobei es ferner eine elektronische Steuer- und Auswerteeinheit umfasst.

## Claims

1. A test system for wound drainage dressings comprising at least one liquid reservoir (8) and a test unit with at least one supply line (181, 184, 191, 194), wherein the said at least one fluid reservoir (8) is connectable with the at least one supply line (181, 184, 191, 194), **characterized in that** the unit comprises:
a main body (1) with at least one cavity (182, 185, 192, 195),
at least one supply line (181, 184, 191, 194),
which runs within the main body (1) and which connects an outer face of the main body (1) to the cavity (182, 185, 192, 195),
a surface of the main body (1), which surface is designed as a support surface (100) for supporting wound dressings (D) and the wound drainage covers thereof, and
several channels (183, 186, 193, 196), which run within the main body (1) and which connect the cavity (182, 185, 192, 195) to the support surface (100),
wherein a vacuum is able to be generated in the cavity (182, 185, 192, 195) and the channels (183, 186, 193, 196) when the support surface (100) is covered in an airtight manner, and that the test system further comprises a drainage container (4), which is connectable to the support surface (100) via a drainage line (3) and a vacuum connection (2).

2. The test system as claimed in claim 1, wherein the main body (1) has several cavities (182, 185, 192, 195), which are connected to at least one outer face of the main body (1) via individual supply lines (181, 184, 191, 194).

3. The test system as claimed in one of claims 1 and 2, wherein the support surface (100) is flat.

4. The test system as claimed in one of claims 1 through 3, wherein several cavities (182, 185, 192, 195) are present that have different volumes.

5. The test system as claimed in one of claims 1 through 4, wherein the channels (183, 186, 193, 196) extend in straight lines in the main body (1).

6. The test system as claimed in one of claims 1 through 5, wherein the channels (183, 186, 193 196) have a constant internal diameter.

7. The test system as claimed in one of claims 1 through 6, wherein the channels (183, 186, 193, 196) assigned to a common cavity (182, 185, 192, 195) have the same internal diameter.

8. The test system as claimed in one of claims 1 through 7, wherein the channels (183, 186, 193, 196) of a common cavity (182, 185, 192, 195) have a different internal diameter than the channels (183, 186, 193, 196) of another cavity (182, 185, 192, 195).

9. The test system as claimed in one of claims 1 through 8, wherein the main body (1) has a base plate (12), a supply plate (10) and, arranged in between these, a sealing plate (11),
wherein the base plate (12) has a plane-parallel configuration and
wherein the supply plate (10) has the following:
- the at least one supply line (181, 184, 191, 194),
- at least one recess for forming the at least one cavity (182, 185, 192, 195), and
- the channels (183, 186, 193, 196).

10. The test system as claimed in one of claims 1 through 9, wherein the at least one supply line (181, 184, 191, 194) runs in a plane perpendicular to the channels (183, 186, 193, 196).

11. The test system as claimed in one of claims 1 through 10, wherein several supply lines (181, 184, 191, 194) are present that open into a common end face of the main body (1).

12. The test system as claimed in one of claims 1 through 11, wherein a vacuum connection (2) is present, which is able to be applied over the support surface (100).

13. The test system as claimed in one of claims 1 through 12, wherein it further comprises a suction pump (6).

14. The test system as claimed in one of claims 1 through 13, wherein it further comprises an electronic control and evaluation unit.

## Revendications

1. Système de vérification de pansements de drainage de blessures, présentant au moins un réservoir de liquide (8) et une unité de vérification qui présente au moins un conduit d'amenée (181, 184, 191, 194), le ou les réservoirs de liquide (8) pouvant être reliés au conduit ou aux conduits d'amenée (181, 184, 191, 194), **caractérisé en ce que** l'unité comporte :
un corps de base (1) doté d'au moins une cavité (182, 185, 192, 195),
au moins un conduit d'amenée (181, 184, 191, 194) qui s'étend dans le corps de base (1) et qui relie un côté extérieur du corps de base (1) à la cavité (182, 185, 192, 195),
une surface du corps de base (1) configurée comme surface de pose (100) qui permet de placer les pansements de blessures (D) et leurs recouvrements de drainage de blessures et
plusieurs canaux (183, 186, 193, 196) qui s'étendent dans le corps de base (1) et qui relient la cavité (182, 185, 192, 195) à la surface de pose (100), une dépression pouvant être formée dans la cavité (182, 185, 192, 195) et dans les canaux (183, 186, 193, 196) lorsque la surface de pose (100) est recouverte de manière étanche à l'air,
le système de vérification présentant en outre un récipient de drainage (4) qui peut être relié à la surface de pose (100) par un conduit de drainage (3) et un raccordement (2) de dépression.

2. Système de vérification selon la revendication 1, dans lequel le corps de base (1) présente plusieurs cavités (182, 185, 192, 195) reliées à au moins un côté extérieur du corps de base (1) par des conduits d'amenée (181, 184, 191, 194) séparés les uns des autres.

3. Système de vérification selon l'une des revendications 1 ou 2, dans lequel la surface de pose (100) a une forme plane.

4. Système de vérification selon l'une des revendications 1 à 3, dans lequel plusieurs cavités (182, 185, 192, 195) sont prévues et présentent des volumes différents.

5. Système de vérification selon l'une des revendications 1 à 4, dans lequel les canaux (183, 186, 193, 196) s'étendent en ligne droite dans le corps de base (1).

6. Système de vérification selon l'une des revendications 1 à 5, dans lequel les canaux (183, 186, 193, 196) présentent un diamètre intérieur constant.

7. Système de vérification selon l'une des revendications 1 à 6, dans lequel les canaux (183, 186, 193, 196) associés à une cavité commune (182, 185, 192, 195) présentent le même diamètre intérieur.

8. Système de vérification selon l'une des revendications 1 à 7, dans lequel les canaux (183, 186, 193, 196) d'une cavité commune (182, 185, 192, 195) présentent un autre diamètre intérieur que les canaux (183, 186, 193, 196) d'une autre cavité (182, 185, 192, 195).

9. Système de vérification selon l'une des revendications 1 à 8, dans lequel le corps de base (1) présente une plaque de base (12) et une plaque d'amenée (10) entre lesquelles est disposée une plaque d'étanchéité (11),
la plaque de base (12) étant plane et la plaque d'amenée (10) présentant les éléments suivants :
- le ou les conduits d'amenée (181, 184, 191, 194),
- au moins une découpe permettant de former la ou les cavités (182, 185, 192, 195) et
- les canaux (183, 186, 193, 196).

10. Système de vérification selon l'une des revendications 1 à 9, dans lequel le ou les conduits d'amenée (181, 184, 191, 194) s'étendent dans un plan perpendiculaire aux canaux (183, 186, 193, 196).

11. Système de vérification selon l'une des revendications 1 à 10, dans lequel plusieurs conduits d'amenée (181, 184, 191, 194) sont prévus et débouchent dans un côté frontal commun du corps de base (1).

12. Système de vérification selon l'une des revendications 1 à 11, dans lequel un raccordement (2) à une dépression est prévu et peut être appliqué au-dessus de la surface de pose (100).

13. Système de vérification selon l'une des revendications 1 à 12, qui comporte en outre une pompe aspirante (6).

14. Système de vérification selon l'une des revendications 1 à 13, qui comporte en outre une unité électronique de commande et d'évaluation.
